# EUROPEAN PATENT APPLICATION

(11) **EP 4 374 809 A1**
(43) Date of publication of application: **29.05.2024**
(21) Application number: 23211438.9
(22) Date of filing: 22.11.2023
(51) Int. Cl.: A61B 18/12, A61B 18/14, A61B 18/00

(54) **COMBINED ELECTRODES FOR TISSUE PENETRATIVE IRREVERSIBLE ELECTROPORATION (IRE)**

(30) Priority: 23.11.2022 US 202217993083
(71) Applicant: Biosense Webster (Israel) Ltd., 2066717 Yokneam (IL)
(72) Inventor: GOVARI, Assaf, 2066717 Yokneam (IL); ALTMANN, Andres Claudio, 2066717 Yokneam (IL); MARZIANO, Lilah, 2066717 Yokneam (IL); SHAMIS, Yuri, 2066717 Yokneam (IL)
(74) Representative: Carpmaels & Ransford LLP

(57) **Abstract**

An irreversible electroporation (IRE) system includes an IRE ablation power source configured to generate bipolar IRE pulses, a switching assembly, and a processor. The switching assembly is configured to short-circuit a first group and a second group of electrodes of a catheter, the groups of electrodes configured to be placed in contact with tissue of organ, so as to create respective combined electrodes of a first size and a second size smaller than the first size, and to connect the IRE ablation power source to the groups of electrodes. The processor is configured to receive target tissue depth of ablation, select the groups of the electrodes, to control the switching assembly to create the combined electrodes and to ablate the tissue by controlling the switching assembly to apply the bipolar IRE pulses to the groups of electrodes to ablate tissue location in contact with a combined electrode to target depth.

## Description

### CROSS-REFERENCE TO RELATED APPLICATIONS

The present application is a continuation-in-part of U.S. Patent Application Publication 2021/0401490 and U.S. Patent Application No. 17/974,738 filed on October 27, 2022.

### FIELD OF THE DISCLOSURE

The present disclosure relates generally to invasive ablation using electrical signals, and particularly to irreversible electroporation (IRE) of cardiac tissue.

### BACKGROUND OF THE DISCLOSURE

Techniques that use a medical probe to perform irreversible electroporation (IRE) of an intra body tissue were previously proposed in the patent literature. For example, PCT International Publication WO 2021/127558 describes devices, systems and methods for treating conditions of the heart, particularly the occurrence of arrhythmias. The devices, systems and methods deliver therapeutic energy to portions the heart to provide tissue modification, such as to the entrances to the pulmonary veins in the treatment of atrial fibrillation. Generally, the tissue modification systems include a specialized catheter, a high voltage waveform generator and at least one distinct energy delivery algorithm.

As another example, U.S. Patent Application Publication 2018/0221078 describes a method of determining a pulsed field ablation waveform parameter for creating a desired lesion characteristic in cardiac tissue. The method of provides an electrosurgical generator configured to deliver electroporation pulses, the generator configured to (i) load predetermined waveform parameters, (ii) load predetermined modeling data, (iii) accept entry of a user inputted desired lesion characteristic, and determine at least one corresponding pulsed field ablation waveform parameter based on (i-iii).

The present disclosure will be more fully understood from the following detailed description of the examples thereof, taken together with the drawings in which:

### BRIEF DESCRIPTION OF THE DRAWINGS

Fig. 1 is a schematic, pictorial illustration of a catheter-based position-tracking and irreversible electroporation (IRE) ablation system, in accordance with an example of the present disclosure;
Figs. 2A and 2B are simulations of IRE electric field strength generated by combined electrodes (2A) vs. single electrodes (2B) of a same catheter using the same potential, in accordance with an example of the disclosure;
Figs. 3A and 3B are simulations of IRE electric field strength generated by electrode pairs held at 1800 V potential difference (2A) vs. 2200 V potential difference (2B), in accordance with an example of the disclosure;
Figs. 4A and 4B are schematic, pictorial illustrations of IRE electric field lines generated by bipolar IRE pulses in successive activations to interleaved subsets of combined electrodes), in accordance with an example of the disclosure;
Fig. 5 is a flow chart that schematically illustrates a method for using combined electrodes for tissue penetrative IRE, in accordance with an example of the disclosure;
Fig. 6 is a schematic, pictorial illustration of IRE electric field lines generated by a bipolar IRE pulse applied between a large combined electrode and a small combined electrode, in accordance with an example of the disclosure; and
Fig. 7 is a flow chart that schematically illustrates a method for using the combined electrodes of Fig. 6 for tissue penetrative IRE, in accordance with an example of the disclosure.

### DETAILED DESCRIPTION OF EXAMPLES

### OVERVIEW

Irreversible electroporation (IRE), also called Pulsed Field Ablation (PFA), is a modality where high electric fields are applied to ablate tissue cells by inducing apoptosis (programmed cell death) in the cells. The IRE fields are typically delivered by a signal generator in the form of bursts of high frequency, high voltage pulses (also called hereinafter a "pulse train"). IRE is typically applied in a bipolar manner, i.e., between pairs of electrodes in contact with tissue, to generate high electric fields (e.g., above a certain threshold) to kill tissue cells between the electrodes.

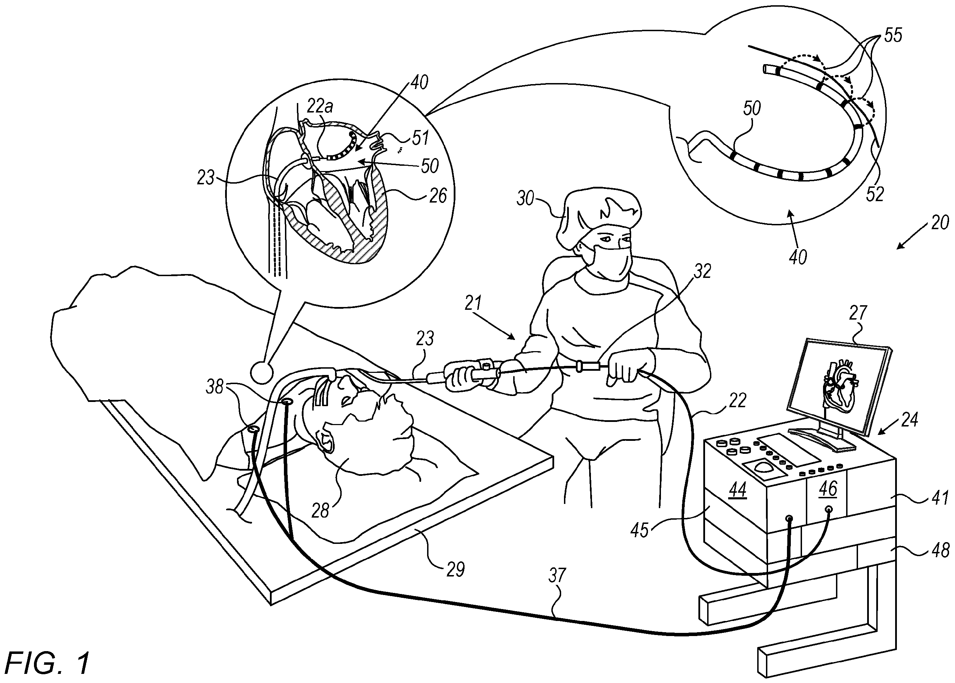

To implement IRE ablation over a relatively large tissue region of an organ, such as a circumference of an ostium of a pulmonary vein (PV), it may be beneficial to use multiple pairs of electrodes of a multi-electrode catheter. Examples of suitable multi-electrode catheters are given in Fig. 1.

To make the generated electric field as spatially uniform as possible over a large tissue region, it may be beneficial to have pairs of electrodes selected with overlapping fields, or at least fields adjacent to each other. However, the ablation causes Joule heating to occur with the IRE generated fields, and this heating may cause unwanted thermal damage to the electrodes and to tissue. The challenge of avoiding Joule heating grows with the necessity to use larger potentials so as to achieve greater tissue depth of ablation. Sufficiently deep lesions are critical for blocking arrhythmogenic pathways in their entirety.

Some examples of the present disclosure that are described hereinafter increase the achievable depth of IRE, with little or no increase in thermal heating, by using a switching assembly configured to short-circuit two or more groups of the electrodes so as to create respective combined electrodes, and to selectively connect the IRE ablation power source to the two or more combined electrodes. To this end, the processor is configured to apply between two or more sets of the electrodes bipolar pulses having an amplitude sufficient to cause IRE in the tissue contacted by the sets of (i.e., combined) electrodes, each set comprising one or more of the electrodes.

The processor is configured to select the two or more groups (e.g., sets) of the electrodes, to control the switching assembly to create the combined electrodes from the selected groups, and to ablate the tissue by controlling the switching assembly to apply the bipolar IRE pulses to pairs of the two or more combined electrodes. Increasing surface area of electrodes by combining such will reduce Joule heating density, and allow thereby local tissue to dissipate the heat more effectively.

The processor is configured to ablate the tissue by controlling the switching assembly to apply the bipolar IRE pulses to pairs of the combined electrodes in order to achieve a given electrical field strength at a given tissue depth.

In some examples, the switching assembly is configured to short-circuit a first group and a second group (252) of electrodes of a catheter, the first group and the second group of electrodes configured to be placed in contact with tissue of an organ, so as to create respective combined electrodes of a first size and a second size smaller than the first size, and to connect the IRE ablation power source to the first group and the second group electrodes. The processor is configured to receive target tissue depth of ablation, select the first group and the second group of the electrodes, to control the switching assembly to create the combined electrodes from the selected groups, and to ablate the tissue by controlling the switching assembly to apply the bipolar IRE pulses to the first group and the second group of electrodes to ablate tissue location in contact with the second combined electrode to a target depth.

To achieve uniform coverage, such as over an entire perimeter of an ostium of a PV, the processor is further configured to apply the bipolar IRE pulses in successive activations between interleaved groups of the combined electrodes.

The disclosed technique therefore controls the depth of ablation (e.g., lesion depth) by selecting a number of electrodes used to create the electric field. Different ablation depths may be achieved as needed. For example, when ablating the ridge of the PV appendage, a depth of 5-6 mm is typically needed. In other areas 3-4 mm may be enough. The processor controls the depth based on the number of electrodes that are used to generate the field. In locations where a shallow depth is desired, each of the V+ and V- potential nodes may be formed with a single electrode. In locations where a deeper depth is desired, each of the V+ and V- nodes may be formed with a pair of electrodes that are short-circuited (to create the aforementioned combined electrode). Shorting the electrodes increases the effective surface area of the electrodes so that more current may be applied to the tissue for ablation without thermal heating.

In some examples, the processor further uses higher voltage and, at the same time, reduces the total number of pulses and bursts (series of pulses) so that the total energy is reduced. By reducing the total energy, thermal heating and bubble formation (a typical outcome of unwanted tissue damage) can be avoided while still achieving deeper depth and spatial uniformity of IRE due to the increase in voltage.

At the same time that the voltages are increased (e.g., by ~20% and ~40%) to make more uniform and deeper penetrating electric fields, the disclosed technique allows for the reduction of the number of pulses per burst in respective steps of ~30%.

Typically, the distance between the electrodes in each pair is the same across all the pairs. By keeping the same inter-electrode distance in each electrode pair, the processor maintains an application of a uniform electric field strength across the lesion as long as the pulses are the same. Inter-electrode distance of a combined electrode pair can range from 0.5 mm to 15 mm (typically 3.5 mm).

The term "electrode pairs" may refer, as with the aforementioned U.S. Patent Application Publication 2021/0401490, to all possible configurations that allow for delivery of biphasic energy between (a) two separate electrodes or (b) between two groups of multiple electrodes. In an example of a basket catheter comprising spines fitted with multiple electrodes each, alternating spines can be energized as one "electrode pair," and alternating interlevel spines energized as a different "electrode pair," and so on in various permutations. In another exmaple, a group of spines acting as one electrode in concert with another group of different spines acting as another electrode for a "pair of electrodes," two or more spines can act as one electrode to operate with two or more spines grouped together as another electrode, and thus to define an "electrode pair" for delivery of biphasic energy to the electrodes. Various permutations of single spines acting as a pair of electrodes can be combined with groups of spines acting as electrode pairs and are considered to be within the scope of the present invention.

In another exmaple, the processor is configured to asymmetrically group the electrodes. For exmaple, assuming a ten-electrode catheter assembly with electrodes e1, e2..., e10, the processor may form a first group with many electrodes (e4-e10 electrodes, or a combined electrode e4-e10) and the second group with few electrodes (e1-e3) and generate the electrical field between. Such electrode arrangement, focuses the ablation at a particular volume near at e1-e3, so that tissue therein can be precisely ablated. Clinically, combined electrode e4-e10 serves as a return electrode with little impact on tissue, due to the weak electrical field therein.

This benefit is achieved since such asymmetrical grouping of the electrodes, causes high concentration of electric filed lines in the second group e1-e3 (i.e., high electric field). This can be very useful when trying to fill in gaps in an ablation line, as filling in gaps typically requires to pinpoint the ablation location. Such asymmetrical grouping of the electrodes can also be useful in preventing collateral damage, such that might be caused to the esophagus or the phrenic nerve.

In some examples a system is provided, that is configured to selectively generate various size and shaped lesions while maintaining Joule heating below a defined threshold. The selective generating is based on controlling: 1. number of short-circuited electrodes; 2. number of pulses per pulse train, and 3. amplitude of the pulses. Thermal heating is controlled by selectively reducing the number of pulses and amplitude to compensate for thermal heating due to shoring electrodes.

Typically, the processor is programmed in software containing a particular algorithm that enables the processor to conduct each of the processor-related steps and functions outlined above.

### SYSTEM DESCRIPTION

Fig. 1 is a schematic, pictorial illustration of a catheter 21 based position-tracking and irreversible electroporation (IRE) ablation system 20, in accordance with an example of the present disclosure. System 20 comprises a deflectable tip section 40 that is fitted at a distal end 22a of a shaft 22 of catheter 21 with deflectable tip section 40 comprising multiple electrodes 50. In the example described herein, electrodes 50 are used for IRE ablation of tissue of a left atrium of heart 26, such as IRE ablation of an ostium 51 tissue of a pulmonary vein in heart 26.

The proximal end of catheter 21 is connected to a control console 24 comprising an IRE power source 45. Console 24 includes a switching box 46 (also referred to as a switching assembly) that can switch to energize any single electrode or electrode pairs among electrodes 50, including using short-circuited electrode pairs (or triplets for that matter) as a single electrode. One or more high voltage IRE ablation protocols of the disclosed method are stored in a memory 48 of console 24.

Ablation source 45, typically a high-voltage signal-generating unit, generates an IRE electric field 55 applied by a portion of electrodes 50 in the form, as one example, of bipolar pulses between pairs of electrodes 50.

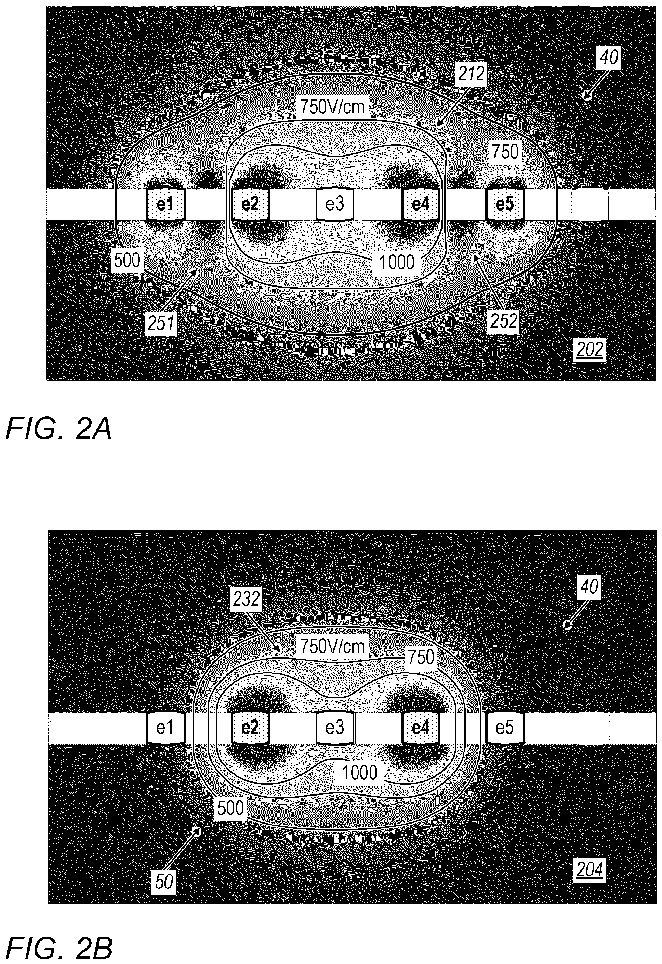

As another example, using the disclosed technique, switching assembly 46 can creates two or more combined electrodes by short-circuiting a portion of electrodes 50, to apply bipolar pulses between one or more pairs of the combined electrodes, as described in Fig. 2. The selection of electrodes 50 aims at achieving uniformity and penetration depth of IRE field 55 into tissue 52 (e.g., of ostium 51) with minimal thermal heating side effects, as further described below in Fig. 2.

Physician 30 inserts distal end 22a of shaft 22 through a sheath 23 into heart 26 of a patient 28 lying on a table 29. Physician 30 navigates the distal end of shaft 22 to a target location in heart 26 by manipulating shaft 22 using a manipulator 32 near the proximal end of the catheter and/or deflection from the sheath 23. During the insertion of distal end 22a, deflectable tip section 40 is maintained in a straightened configuration by sheath 23. By containing tip section 40 in a straightened configuration, sheath 23 also serves to minimize vascular trauma along the way to target location.

Once distal end 22a of shaft 22 has reached the target location, physician 30 retracts sheath 23 and deflects tip section 40, and further manipulates shaft 22 to place electrodes 50 disposed over tip section 40 in contact with ostium 51 the pulmonary vein.

Electrodes 50 are connected by wires running through shaft 22 to processor 41 controlling switching box 46 of interface circuits 44 in a console 24.

In an example, processor 41 receives electrical impedance signals, measured between electrodes 50 and surface electrodes 38, which are seen in the exemplified system as attached by wires running through a cable 37 to the chest of patient 28. A method for tracking the positions of electrodes 50 using the measured impedances is implemented in various medical applications, for example in the CARTO^{™} system, produced by Biosense Webster, Inc. (Irvine, California) and is described in detail in U.S. Patent 8,456,182, which is assigned to the assignee of the current disclosure. This method is sometimes called Advanced Catheter Location (ACL). Console 24 drives a display 27, which shows the tracked position and/or shape of deflectable tip section 40 inside heart 26.

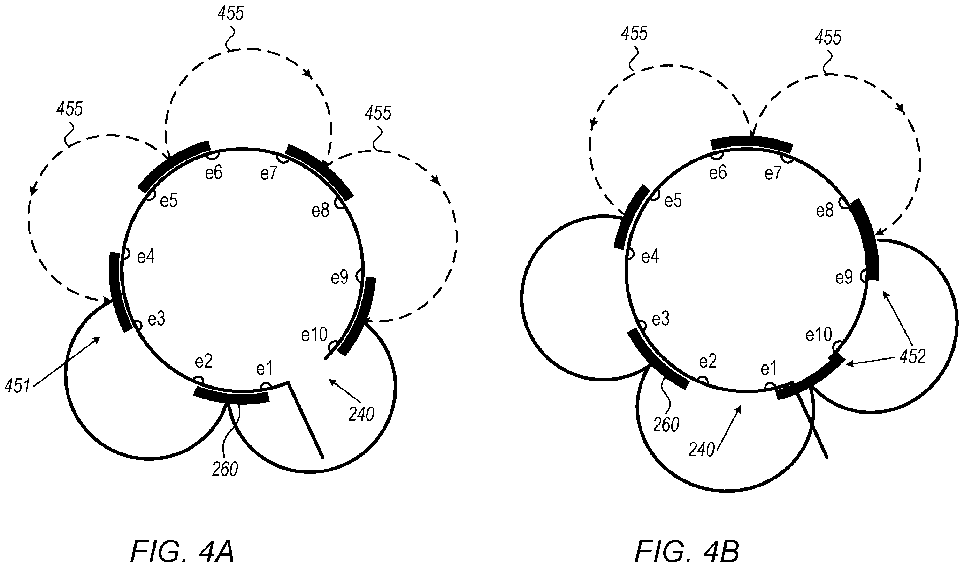

Processor 41, shown comprised in control console 24, is typically a general-purpose computer, with suitable front end and interface circuits 44 for receiving signals from catheter 21, as well as for applying RF energy treatment via catheter 21 in a left atrium of heart 26 and for controlling the other components of system 20. Processor 41 typically comprises software in a memory 48 of system 20 that is programmed to carry out the functions described herein. The software may be downloaded to the computer in electronic form, over a network, for example, or it may, alternatively or additionally, be provided and/or stored on non-transitory tangible media, such as magnetic, optical, or electronic memory. In particular, processor 41 runs a dedicated algorithm as disclosed herein, included in Fig. 4, that enables processor 41 to perform the disclosed steps, as further described below.

The disclosed high-voltage IRE ablation method applies to many types of multi-electrode catheters, including expendable-frame such as basket catheters.. Catheters of other shapes can also be used with the disclosed technique, such those having deflectable tips disposed with a one-dimensional array of electrodes, flat catheters disposed with a two-dimensional array of electrodes, or basket catheters. The electrodes themselves may have any shape suitable for bipolar IRE ablation, e.g., flat or ring.

### USING COMBINED ELECTRODES FOR IRE

Figs. 2A and 2B are simulations (202, 204) of IRE electric field strength generated by combined electrodes (2A) vs. single electrodes (2B) of a same catheter using a same potential, in accordance with an example of the disclosure. Fig. 2 shows a portion of deflectable tip section 40, which can be a portion of the aforementioned Lasso^{™} catheter, disposed with electrodes 50. Simulations 202 and 204 compare the extent of the electric field (e.g., field depth) when applying the same voltage in two cases:
A. The voltage is induced between short-circuited electrodes e1 and e2 (i.e., combined electrode C1=e12) and short-circuited electrodes e4 and e5 (i.e., combined electrode C4=e45).
B. The voltage is induced between electrode e1 and e3.

As seen, by comparing the 750 V/cm field lines 212 (2A) and 232 (2B), two combined electrodes, as compared to two standalone electrodes, achieves deeper penetration for the same voltage. In particular, using combined electrodes C1 251 and C4 252 achieves significantly more depth of the 750 V/cm field at the location of e3.

### EFFECT OF VOLTAGE INCREASE ON IRE WITH COMBINED ELECTRODES

As noted above, the disclosed technique can utilize electrode pairs held at higher voltage difference and, at the same time, reduce the total number of pulses and bursts (series of pulses) so that the total energy is reduced.

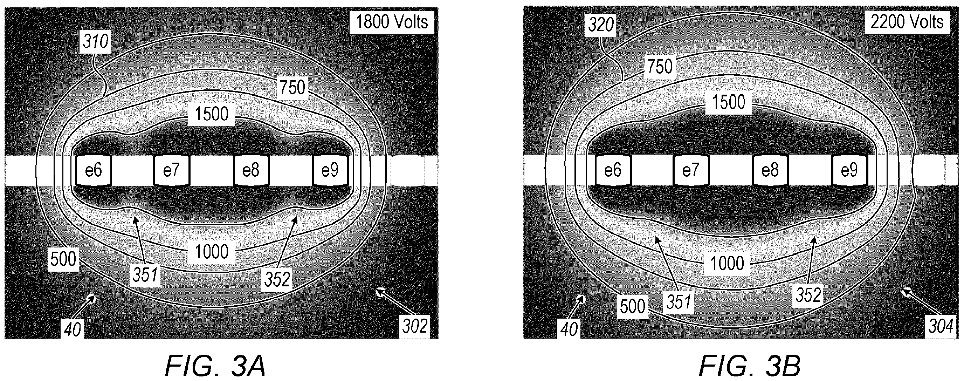

Figs. 3A and 3B are simulations of IRE electric field strength generated by an electrode pair held at 1800 V potential difference (2A) vs. 2200 V potential difference (2B), in accordance with an example of the disclosure.

In Figs 3A and 3B, a portion of deflectable tip section 40 is again seen with electrodes C6=e67 (351) and C8=e89 (352) .

Numerical simulations 302 and 304 of Fig. 3 compare, by way of example, IRE electric field depth in millimeters, when given voltages of 1800 V (3a) and 2220 V (3B) are applied between combined electrodes C6 351 and C8 352. As seen, in 1800 V, an electric field of 750 V/cm reaches to a depth of approximately ~5 mm (310), while, when the voltage is increased by ~20%, e.g., to 2200 V, the same field strength reaches to a depth of ~8 mm (320).

In some cases, the disclosed technique using combined electrodes of a larger effective area than single electrodes, and hence better thermal behavior, may be utilized to further increase the voltage, e.g., by further ~20%, up to 2600 V, without thermal hazards involved with smaller electrodes. One way to reduce thermal hazard is to maintain power, where the disclosed technique allows, and, at the same time, to increase voltage, to reduce the number of pulses per burst in respective steps of ~30%, e.g., from about 20 to about 14 and with a further voltage increase from about 14 to less than 10.

As used herein, the term "approximately" for any numerical values or ranges indicates a suitable dimensional tolerance that allows a part or a collection of components to function for its intended purpose as described herein. More specifically, "approximately" may refer to the range of values ±20% of the recited value, e.g., "approximately 90%" may refer to the range of values from 71% to 99%.

### IRE WITH INTERLEAVED SUBSETS OF COMBINED ELECTRODES

Figs. 4A and 4B are schematic, pictorial illustrations of IRE electric field lines generated by bipolar IRE pulses in successive activations to interleaved subsets (e.g., groups) of combined electrodes (451, 452), in accordance with an example of the disclosure. Combined electrodes are achieved by achieved by short-circuiting (schematically represented by curves 260) of individual electrodes.

Figs. 4A and 4B show a distal end assembly 240 of a Lasso^{™} catheter, which is disposed with ten electrodes, e1, e2,..., e10.

In an example, the pulses are gated to be applied synchronously with the beating of the heart, i.e., to be applied during a refractory period of the tissue.

By way of example, the IRE pulse applied at each heart beat cycle may be specified by the following Table I (achieved by short-circuiting 260 of electrodes (short-circuited electrodes ei and ej denoted as "eij") to create the combined electrodes listed below). In the table, the potential is between combined electrodes Ci and Cⱼ, where:
C1= e12, C2= e23, C3= e34, C4= e45, C5= e56, C6= e67, C7= e78, C9= e910, C10= e101.

**Table I**

| **Parameter** | **Value** |
|---|---|
| Combined electrode cycle 1 | C1, C3, C5 |
| Combined electrode cycle 2 | C2, C4, C6 |
| Combined electrode cycle 3 | C7, C9 |
| Combined electrode cycle 4 | C8, C10 |
| Preset IRE peak voltage | 1800 V |
| Pulse width | 5 microseconds |
| Number of pulses in train | 14 |

As seen in Table I, the processor applies the bipolar IRE pulses in successive activations (for a total of four cycles) to interleaved subsets {e.g., (C1, C3, C5) and (C2, C4, C6)) of the combined electrodes to achieve full coverage. In another example, physician 30 adjusts (e.g., increases) the IRE peak voltage of Table I to 2200 V and reduces the number of pulses in train to 10, for tissue penetration and thermal considerations described above.

As seen in Fig. 4, the application of the protocol generates two subsets of interleaved electric field lines 455, which cover an entire circumference. Such a mode of IRE can be used in isolation of an ostium of a PV.

The pictorial side view shown in Fig. 4 is chosen by way of example, where other protocols are possible. For example, in another example, bipolar voltages applied between two next-adjacent electrodes (i.e., every third electrode, e.g., between combined electrodes C1, C4, C7, C10 in cycle 1 and between combined electrodes C2, C5, C8, in cycle 2, and C9, C2 in cycle 3).

### METHOD OF IRE WITH INTERLEAVED SUBSETS OF COMBINED ELECTRODES

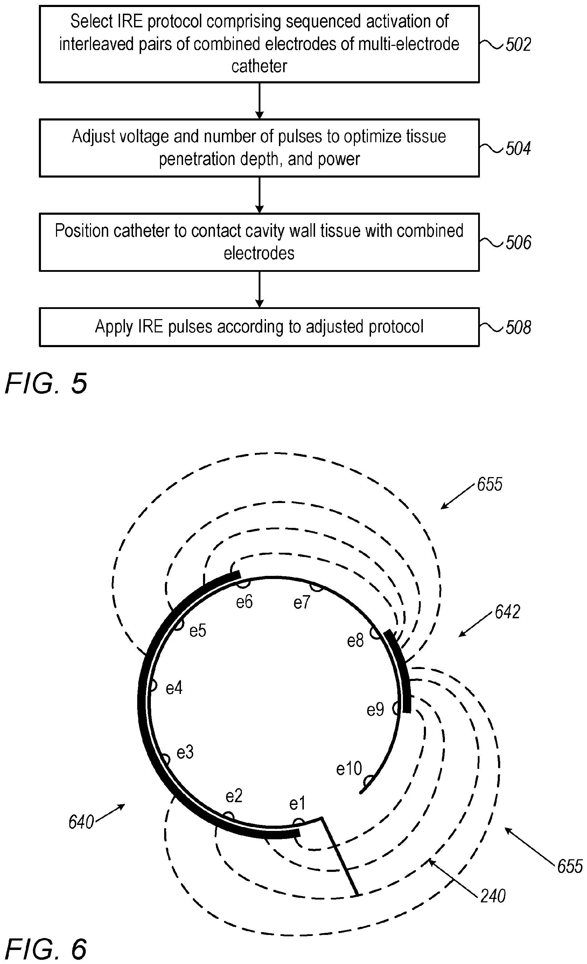

Fig. 5 is a flow chart that schematically illustrates a method for using combined electrodes for tissue penetrative IRE, in accordance with an example of the disclosure. The algorithm, according to the presented example, carries out a process that begins at an IRE protocol selection step 502, when physician 30 selects an IRE protocol comprising sequenced activation of interleaved pairs of combined electrodes of a multi-electrode catheter, such as pairs of combined electrodes of catheter 21 seen in Figs 4A and 4B. An example protocol is provided above by Table I.

Next, at protocol adjustment step 504, physician 30 adjusts (e.g., increases) the IRE peak voltage to 2200 V and reduces the number of pulses in train to 10, for tissue penetration and thermal considerations described above.

Next, physician 30 inserts, navigates, and positions the catheter at a target location within a cavity of an organ of patient, such as at ostium 51, at a catheter positioning step 506. In particular, physician 30 makes sure that the combined electrodes are in contact with wall tissue.

Finally, physician 30 uses system 20, and the adjusted IRE protocol, to apply IRE pulses according to the sequence specified in the protocol (e.g., according to the sequence of Table I), to energize each subset of the combined electrode pairs, at a sequenced IRE ablation step 508.

The example flow chart shown in Fig. 5 is chosen purely for the sake of conceptual clarity. In alternative examples, additional steps may be performed, such as processor 41 monitoring measured temperature of electrodes, and acting according to measured temperatures, if required, such as disconnecting an overheated electrode pair from further use in the specified protocol.

### ASYMMETRICAL GROUPING OF THE ELECTRODES

Fig. 6 is a schematic, pictorial illustration of IRE electric field lines 655 generated by a bipolar IRE pulse applied between a large combined electrode 640 and a small combined electrode 642, in accordance with an example of the disclosure. Fig. 6 shows distal end assembly 240 of a Lasso^{™} catheter, which is disposed with ten electrodes, e1, e2,..., e10.

As seen, the processor forms a first group with many electrodes (e1-e6 electrodes, or a combined electrode e1-e6) and the second group with fewer electrodes (e8-e9) and generate the electrical field between. Such electrode arrangement, focuses the ablation at a particular volume near e8-e9 surface so that tissue therein can be precisely ablated. Clinically, combined electrode e4-e10 serves as a return electrode with little impact on tissue, due to the weak electrical field therein.

This benefit is achieved since such asymmetrical grouping of the electrodes, causes high concentration of electric filed lines in the second group e8-e9 (i.e., high electric field).

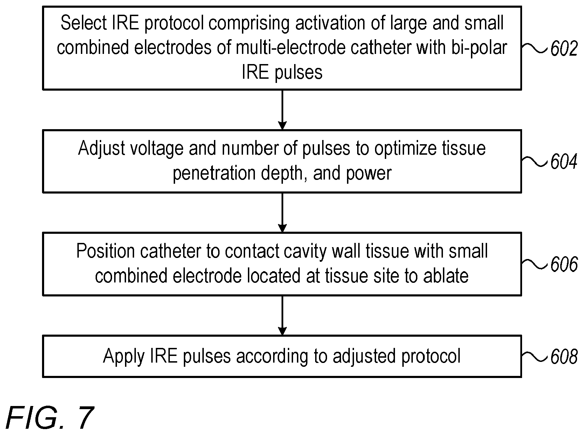

Fig. 7 is a flow chart that schematically illustrates a method for using the combined electrodes of Fig. 6 for tissue penetrative IRE, in accordance with an example of the disclosure.

The algorithm, according to the presented example, carries out a process that begins at an IRE protocol selection step 702, when physician 30 selects an IRE protocol comprising activation with bipolar IRE pulses of two combined electrodes, one large and one small, of a multi-electrode catheter, such as pairs of combined electrodes of catheter 21 seen in Fig. 6.

Next, at protocol adjustment step 704, physician 30 adjusts (e.g., increases) the IRE peak voltage to 2200 V and reduces the number of pulses in train to 10, for tissue penetration and thermal considerations described above.

Next, physician 30 inserts, navigates, and positions the catheter at a target location within a cavity of an organ of patient, such as at ostium 51, at a catheter positioning step 706. In particular, physician 30 makes sure that the combined electrodes are in contact with wall tissue, with the small combined electrode located at the tissue site to ablate.

Finally, physician 30 uses system 20, and the adjusted IRE protocol, to apply IRE pulses according to the sequence specified in the protocol (e.g., according to the sequence of Table I), to energize each subset of the combined electrode pairs, at IRE ablation step 708.

The example flow chart shown in Fig. 7 is chosen purely for the sake of conceptual clarity. In alternative examples, additional steps may be performed, such as processor 41 monitoring measured temperature of electrodes, and acting according to measured temperatures, if required, such pausing ablation.

### EXAMPLES

### Example 1

An irreversible electroporation (IRE) system (20) includes an IRE ablation power source (45), a switching assembly (46), and a processor (41). The IRE ablation power source is configured to generate bipolar IRE pulses. The switching assembly is configured to short-circuit a first group and a second group of electrodes of a catheter, the first group and the second group of electrodes configured to be placed in contact with tissue (52) of an organ (26), so as to create respective combined electrodes (640, 642) of a first size and a second size smaller than the first size, and to connect the IRE ablation power source to the first group and the second group electrodes. The processor is configured to receive target tissue depth of ablation, select the first group and the second group of the electrodes, to control the switching assembly to create the combined electrodes from the selected groups, and to ablate the tissue by controlling the switching assembly to apply the bipolar IRE pulses to the first group and the second group of electrodes to ablate tissue location in contact with the second combined electrode to a target depth.

### Example 2

The system according example 1, wherein the processor (41) is further configured to increase the voltage of the bipolar IRE pulses, while at a same time reduce number of pulses per burst.

### Example 3

The system according to any of examples 1 and 2, wherein the processor (41) is further configured to monitor a measured temperature of the small combined electrode (642), and act according to measured temperature.

### Example 4

The system according to any of examples 1 through 3, wherein the tissue (52) comprises cardiac tissue, and wherein the processor (41) is configured to gate the bipolar IRE pulses to synchronize with refractory periods of the cardiac tissue.

### Example 5

An irreversible electroporation (IRE) system (20) includes an IRE ablation power source (45), a switching assembly (46), and a processor (41). The IRE ablation power source is configured to generate bipolar IRE pulses. The switching assembly is configured to short-circuit two or more groups of electrodes of a catheter, the multiple electrodes configured to be placed in contact with tissue of an organ, so as to create respective combined electrodes (251, 252), and to selectively connect the IRE ablation power source to the two or more combined electrodes. The processor is configured to receive target tissue depth of ablation select the two or more groups of the electrodes, to control the switching assembly to create the combined electrodes from the selected groups, and to ablate the tissue by controlling the switching assembly to apply the bipolar IRE pulses to pairs of the two or more combined electrodes to ablate tissue therein to the target depth.

### Example 6

The system according to examples 5, wherein the processor is further configured to apply the bipolar IRE pulses in successive activations to interleaved subsets (451, 452) of the combined electrodes.

### Example 7

An irreversible electroporation (IRE) method includes generating bipolar IRE pulses using an IRE ablation power source (45). Using a switching assembly (46), a first group and a second group of electrodes of a catheter are short-circuited, the first group and the second group of electrodes configured to be placed in contact with tissue (52) of an organ, so as to create respective combined electrodes (640, 642) of a first size and a second size smaller than the first size. The IRE ablation power source is connected to the first group and the second group electrodes. In a processor (41), upon receiving target tissue depth of ablation, the first group and the second group of the electrodes are selected, to control the switching assembly to create the combined electrodes from the selected groups. Tissue is ablated by controlling the switching assembly to apply the bipolar IRE pulses to the first group and the second group of electrodes to ablate tissue location in contact with the second combined electrode to a target depth.

### Example 8

An irreversible electroporation (IRE) method includes generating bipolar IRE pulses using an IRE ablation power source (45). Using a switching assembly (46), two or more groups of electrodes of a catheter are short-circuited, the multiple electrodes configured to be placed in contact with tissue of an organ, so as to create respective combined electrodes (251, 252). The IRE ablation power source is selectively connected to the two or more combined electrodes. In a processor (41), upon receiving target tissue depth of ablation, the two or more groups of the electrodes are selected, to control the switching assembly to create the combined electrodes from the selected groups. The tissue is ablated by controlling the switching assembly to apply the bipolar IRE pulses to pairs of the two or more combined electrodes to ablate tissue therein to the target depth.

Although the examples described herein mainly address pulmonary vein isolation, the methods and systems described herein can also be used in other applications that may require a sequenced ablation, such as, for example, in renal denervation, and generally, in ablating other organs, such as in treatment of lung or liver cancers.

It will thus be appreciated that the examples described above are cited by way of example, and that the present disclosure is not limited to what has been particularly shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. An irreversible electroporation (IRE) system (20), comprising:
an IRE ablation power source (45) configured to generate bipolar IRE pulses;
a switching assembly (46) which is configured to short-circuit a first group and a second group of electrodes of a catheter, the first group and the second group of electrodes configured to be placed in contact with tissue (52) of an organ (26), so as to create respective combined electrodes (640, 642) of a first size and a second size smaller than the first size, and to connect the IRE ablation power source to the first group and the second group electrodes; and
a processor (41), which is configured to receive target tissue depth of ablation, select the first group and the second group of the electrodes, to control the switching assembly to create the combined electrodes from the selected groups, and to ablate the tissue by controlling the switching assembly to apply the bipolar IRE pulses to the first group and the second group of electrodes to ablate tissue location in contact with the second combined electrode to a target depth.

2. The system according to claim 1, wherein the processor (41) is further configured to increase the voltage of the bipolar IRE pulses, while at a same time reduce number of pulses per burst.

3. The system according to claim 1 or claim 2, wherein the processor (41) is further configured to monitor a measured temperature of the small combined electrode (642), and act according to measured temperature.

4. The system according to any preceding claim, wherein the tissue (52) comprises cardiac tissue, and wherein the processor (41) is configured to gate the bipolar IRE pulses to synchronize with refractory periods of the cardiac tissue.

5. An irreversible electroporation (IRE) system (20), comprising:
an IRE ablation power source (45) configured to generate bipolar IRE pulses;
a switching assembly (46) which is configured to short-circuit two or more groups of electrodes of a catheter, the multiple electrodes configured to be placed in contact with tissue of an organ, so as to create respective combined electrodes (251, 252), and to selectively connect the IRE ablation power source to the two or more combined electrodes; and
a processor (41), which is configured to receive target tissue depth of ablation select the two or more groups of the electrodes, to control the switching assembly to create the combined electrodes from the selected groups, and to ablate the tissue by controlling the switching assembly to apply the bipolar IRE pulses to pairs of the two or more combined electrodes to ablate tissue therein to the target depth.

6. The system according to claim 5, wherein the processor is further configured to apply the bipolar IRE pulses in successive activations to interleaved subsets (451, 452) of the combined electrodes.

7. An irreversible electroporation (IRE) method, comprising:
generating bipolar IRE pulses using an IRE ablation power source (45);
using a switching assembly (46), short-circuiting a first group and a second group of electrodes of a catheter, the first group and the second group of electrodes configured to be placed in contact with tissue (52) of an organ, so as to create respective combined electrodes (640, 642) of a first size and a second size smaller than the first size, and connecting the IRE ablation power source to the first group and the second group electrodes; and
in a processor (41), upon receiving target tissue depth of ablation, selecting the first group and the second group of the electrodes, to control the switching assembly to create the combined electrodes from the selected groups, and ablating the tissue by controlling the switching assembly to apply the bipolar IRE pulses to the first group and the second group of electrodes to ablate tissue location in contact with the second combined electrode to a target depth.

8. The method according to claim 7, and comprising increasing the voltage of the bipolar IRE pulses, while at a same time reducing number of pulses per burst.

9. The method according to claim 7, and comprising monitoring a measured temperature of the small combined electrode, and acting according to measured temperature.

10. The method according to claim 7, wherein the tissue comprises cardiac tissue, and comprising gating the bipolar IRE pulses to synchronize with refractory periods of the cardiac tissue.

11. An irreversible electroporation (IRE) method, comprising:
generating bipolar IRE pulses using an IRE ablation power source (45);
using a switching assembly (46), short-circuiting two or more groups of electrodes of a catheter, the multiple electrodes configured to be placed in contact with tissue of an organ, so as to create respective combined electrodes (251, 252), and to selectively connecting the IRE ablation power source to the two or more combined electrodes; and
in a processor (41), upon receiving target tissue depth of ablation, selecting the two or more groups of the electrodes, to control the switching assembly to create the combined electrodes from the selected groups, and ablating the tissue by controlling the switching assembly to apply the bipolar IRE pulses to pairs of the two or more combined electrodes to ablate tissue therein to the target depth.

12. The method according to claim 11, and comprising applying the bipolar IRE pulses in successive activations to interleaved subsets of the combined electrodes.
